# EUROPÄISCHE PATENTSCHRIFT

(11) **EP 2 991 696 B1**
(45) Veröffentlichungstag und Bekanntmachung des Hinweises auf die Patenterteilung: **05.07.2017**
(21) Anmeldenummer: 14720594.2
(22) Anmeldetag: 29.04.2014
(51) Int. Cl.: A61M 1/00

(54) **FLÜSSIGKEITSAUFNAHMEBEHÄLTER FÜR EINE VORRICHTUNG ZUR BEREITSTELLUNG VON UNTERDRUCK FÜR MEDIZINISCHE ANWENDUNGEN, VERFAHREN ZUR HERSTELLUNG DES BEHÄLTERS, SOWIE VORRICHTUNG**
LIQUID-ACCOMMODATING CONTAINER FOR A DEVICE FOR PROVIDING VACUUM FOR MEDICAL APPLICATIONS, METHOD FOR PRODUCING THE CONTAINER, AND DEVICE
RÉCIPIENT RÉCEPTEUR DE LIQUIDE POUR UN DISPOSITIF DE PRODUCTION DE VIDE DESTINÉ À DES APPLICATIONS MÉDICALES, PROCÉDÉ DE FABRICATION DU RÉCIPIENT ET DISPOSITIF

(30) Priorität: 03.05.2013 DE 102013208108
(43) Veröffentlichungstag der Anmeldung: 09.03.2016
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); KIRSTEN, Juana, 8488 Turbenthal (CH); FONFARA-DOERR, Astrid, 89077 Ulm (DE); MAHLER, Andreas, 89143 Blaubeuren-Asch (DE)
(74) Vertreter: DREISS Patentanwälte PartG mbB
(86) Internationale Anmeldenummer: PCT/EP2014/058691
(87) Internationale Veröffentlichungsnummer: WO 2014/177545

(56) Entgegenhaltungen:
- WO-A1-96/05873
- WO-A1-2008/100438
- DE-A1-102009 038 130
- US-A1- 2010 016 816

## Beschreibung

Die Erfindung betrifft einen Flüssigkeitsaufnahmebehälter für eine am Körper eines Benutzers tragbare und zusätzlich stationär betreibbare Vorrichtung zur Bereitstellung von Unterdruck für medizinische Anwendungen, insbesondere zur Unterdruckbehandlung von Wunden am menschlichen oder tierischen Körper, wobei die Vorrichtung einen ersten Gehäuseteil mit einer Unterdruck erzeugenden Einrichtung und den nach Gebrauch wegwerfbaren Behälter zur Aufnahme von Körperflüssigkeiten in seinem Inneren, insbesondere von aus einer Wunde abgesaugten Wundsekreten, umfasst, wobei der Behälter einen zweiten Gehäuseteil der Vorrichtung bildet und an dem ersten Gehäuseteil der Vorrichtung manuell befestigbar und manuell lösbar ist und im befestigten Zustand das Innere des Behälters von der Unterdruck erzeugenden Einrichtung mit Unterdruck beaufschlagbar ist, und wobei am Behälter ein Anschluss für eine zum Körper führende Saugleitung vorgesehen ist, so dass eine Unterdruckkommunikation zwischen der Unterdruck erzeugenden Einrichtung, dem Behälter und der zum Körper führenden Saugleitung herstellbar ist, wobei der Behälter zwei Halbschalenteile umfasst, die gegeneinander unlösbar gefügt sind und dabei das Innere des Behälters begrenzen, wobei auch ein Bereich zur Aufnahme eines Filters im Inneren des Behälters gebildet wird.

Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 250 mmHg (mm Quecksilbersäule) unterhalb des umgebenden Atmosphärendrucks liegt. Es hat sich gezeigt, dass dies der Wundheilung förderlich ist. Für den unterdruckdichten Abschluss wird typischerweise ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann.

Der vorliegenden Erfindung liegt die Aufgabe zugrunde, die Aufnahme und Montage des Filters auf wirtschaftliche Weise und zudem betriebssicher zu gestalten.

Diese Aufgabe wird bei einem Flüssigkeitsaufnahmebehälter der genannten Art erfindungsgemäß dadurch gelöst, dass der Filter in einer axialen Richtung, die ungefähr senkrecht zu einer Teilungsebene der Halbschalenteile verläuft, einerseits gegen einen Innenbereich des vom ersten Gehäuseteil abgewandten Halbschalenteils und andererseits gegen ein Dichtungselement unter axialer Spannung anliegt, welches Dichtungselement an das dem ersten Gehäuseteil zugewandte Halbschalenteil angefügt ist, wobei die axiale Spannung durch Zusammenfügen der Halbschalenteile und damit einhergehender Verformung des Dichtungselements durch den Filter gebildet ist.

Dadurch, dass der Filter unter axialer Spannung zwischen den beiden Halbschalenteilen aufgenommen ist, ist zum einen eine sichere Lagefixierung des Filters innerhalb des Behälters erreicht. Durch den axialen Druck wird auch eine sichere Abdichtung zwischen dem Filter und dem Dichtungselement erreicht. Es können auch Maßtoleranzen bei dem Filter ausgeglichen werden oder trotz Maßtoleranzen eine sichere Abdichtung erreicht werden. Die Montage des Filters gestaltet sich sehr einfach, da der Filter entweder schon vor dem Zusammenfügen der Halbschalenteile zwischen diesen angeordnet werden kann. Es wäre aber auch denkbar, dass der Filter durch eine entsprechend groß gestaltete Durchgangsöffnung in einer Wandung des dem ersten Gehäuseteil zugewandten Halbschalenteils des Behälters in den Behälter eingesetzt und dann durch Anordnen der Dichtung in dieser Durchgangsöffnung lagefixiert wird.

In vorteilhafter Weiterbildung der Erfindung liegt der Filter gegen vorzugsweise mehrere Rippen an, die im Innenbereich des von dem ersten Gehäuseteil abgewandten Halbschalenteils ausgebildet sind. Gegenüber einer flächenhaften Anlage erweist sich dies als vorteilhaft, weil hierdurch eine größere aktive Filterfläche gegenüber dem übrigen Innenraum, also dem Flüssigkeitssammelraum des Behälters, exponiert werden kann.

Der Filter selbst kann beispielsweise block- oder scheibenförmig ausgebildet sein. Indessen erweist es sich als vorteilhaft, wenn der Filter zylindrisch, topf- oder konus- oder kegelmantelförmig ausgebildet ist, da hierdurch eine größere Durchdringungsfläche des Filters zur Verfügung steht. Ungeachtet der konkreten Gestalt des Filters ist er vorteilhafterweise aus einem starren und formstabilen Material ausgebildet. Als Filtermaterial geeignet sind beispielsweise poröse Feststoffe aus Kunststoff (beispielsweise aus Polyethylen oder Polypropylen) oder aus Keramik oder Metall.

Weiterhin kann eine Beimischung einer quellbaren Substanz (beispielsweise Carboxymethylzellulose) vorhanden sein, welche nach Kontakt mit Flüssigkeit die im Filtermaterial vorhandenen Poren verschließt und so einen weiteren Flüssigkeitsdurchtritt durch den Filter verhindert. Vorteilhaft ist auch die Beimischung einer geruchsabsorbierenden Substanz (beispielsweise Aktivkohle), welche gasförmige Stoffe, die eine Geruchsbelästigung verursachen können, zurückhält. Die geruchsabsorbierende Substanz kann auch als Beschichtung vorhanden sein.

Der Filter ist vorteilhafterweise in einer Kammer aufgenommen, die von einer auf sich selbst zurückgeführten Wandung im Inneren des Behälters begrenzt ist. Diese Wandung kann in vorteilhafter Weise eine oder mehrere Durchgangsöffnungen aufweisen, mittels derer sie mit dem übrigen Inneren des Behälters oder mit einer spritzwassergeschützten Vorkammer in Strömungsverbindung steht.

Weiter erweist es sich als vorteilhaft, wenn das Dichtungselement ein Spritzgussteil aus einem elastisch nachgiebigen Material ist, welches in einer Durchgangsöffnung in einer Wandung des ersten Halbschalenteils des Behälters vorzugsweise formschlüssig gehalten ist. Das Dichtungselement kann beispielsweise angeklebt oder eingeklipst oder mittels weiterer Montageelemente in seiner Betriebsposition fixiert sein.

In Weiterbildung der Erfindung erweist es sich als vorteilhaft, wenn das Dichtungselement ein Spritzgussteil aus einem elastisch nachgiebigen Material ist, welches in einer Durchgangsöffnung in einer Wandung des ersten Halbschalenteils des Behälters angeordnet und an die Wandung angespritzt ist. Beim Anspritzen des Dichtungselements kann zugleich ein formschlüssiger Hintergriff realisiert werden.

Bei einer bevorzugten Ausführungsform der Erfindung weist das Dichtungselement eine axiale Durchgangsöffnung auf.

Weiter erweist es sich als vorteilhaft, wenn das Dichtungselement bezüglich der axialen Richtung konzentrisch ausgebildet ist.

Weiter erweist es sich als vorteilhaft, wenn das Dichtungselement eine Abstufung bezüglich der axialen Richtung aufweist, gegen die der Filter anliegt und die vorzugsweise radial innen ausgebildet ist.

Weiter erweist es sich als vorteilhaft, wenn das Dichtungselement einen in das Innere des Behälters vorstehenden rohrförmigen Ansatz aufweist, in den der Filter eingreift. Diese Ausführungsform hat den Vorteil, dass der Filter während der Montage in den rohrförmigen Ansatz eingesteckt werden kann, so dass er hierdurch in seiner korrekten Montageposition, insbesondere durch Reibschluss, positionierbar ist.

Weiter erweist es sich als vorteilhaft, wenn das Dichtungselement einen von dem Behälter in Richtung auf das erste Gehäuseteil vorstehenden rohrförmigen Ansatz aufweist, mittels dessen eine Strömungsverbindung zu der Unterdruck erzeugenden Einrichtung herstellbar ist. Das Dichtungselement übernimmt also eine duale Funktion: Es dichtet den Filter in dem Behälter nach außen ab, und es bildet zugleich eine Schnittstelle zur Strömungskommunikation zu der Unterdruck erzeugenden Einrichtung in dem ersten Gehäuseteil.

In weiterer Ausbildung der Erfindung wird vorgeschlagen, dass ein weiteres Dichtungselement vorgesehen ist, welches zwischen einer insbesondere abgeschlossenen Kammer im Inneren des Behälters und einem Medien- oder Messanschluss in dem ersten Gehäuseteil angeordnet ist und von dem Behälter in Richtung auf das erste Gehäuseteil mit einem rohrförmigen Ansatz vorsteht. Dieses weitere Dichtungselement kann grundsätzlich in derselben Weise hergestellt und angeordnet werden wie das besagte dem Filter zugeordnete Dichtungselement. Es kann insbesondere direkt an eine Wandung des Behälters angespritzt sein.

Hierbei kann es sich als vorteilhaft erweisen, wenn die Dichtungselemente im selben Spritzgießvorgang ausgebildet sind und bei dem Behälter über eine mit elastomerem Material gefüllte Füllstrecke verbunden sind.

Des Weiteren ist Gegenstand der Erfindung ein Verfahren zum Herstellen eines Behälters mit den Merkmalen des Anspruch 15 und eine Vorrichtung mit den Merkmalen des Anspruchs 16.

Weitere Einzelheiten, Merkmale und Vorteile der Erfindung ergeben sich aus den beigefügten Patentansprüchen und aus der zeichnerischen Darstellung und nachfolgenden Beschreibung einer Ausführungsform der Erfindung.

In der Zeichnung zeigt:
Figuren 1a, b eine perspektivische Ansicht einer Vorrichtung zur Bereitstellung von Unterdruck für medizinische Anwendungen mit einem nach Gebrauch wegwerfbaren Behälter zur Aufnahme von Körperflüssigkeiten;
Figur 2 eine perspektivische Ansicht einer Halbschale des Behälters nach Figur 1 von innen gesehen;
Figur 3 eine perspektivische Ansicht der Halbschale nach Figur 2 von außen betrachtet;
Figur 4a die Halbschale nach Figur 3 mit angespritzten Dichtungselementen;
Figuren 4b, c zwei Ansichten der angespritzten Dichtungselemente;
Figur 5 die andere Halbschale des Behälters nach Figur 1 von innen gesehen;
Figur 6 die Halbschale nach Figur 5 von außen gesehen und
Figuren 7a, b Schnittansichten des Behälters.

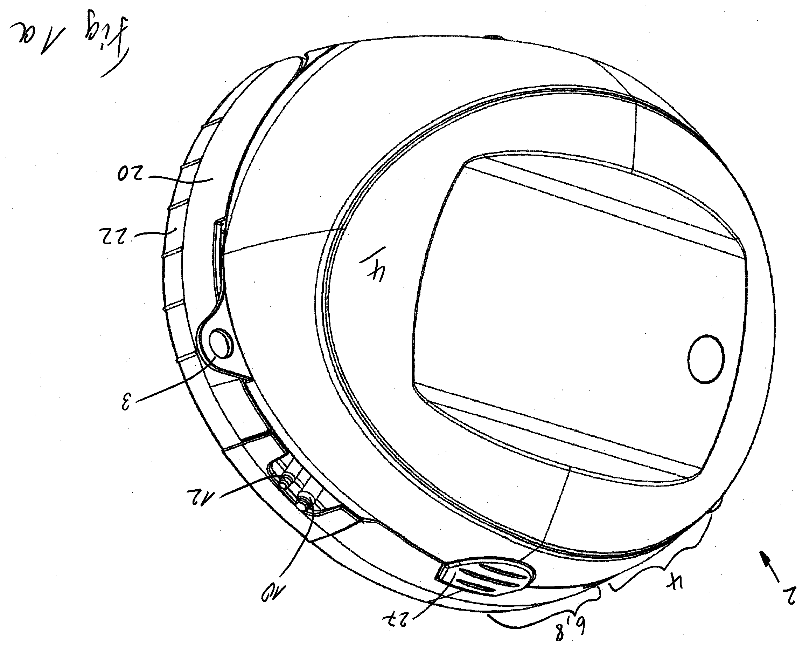

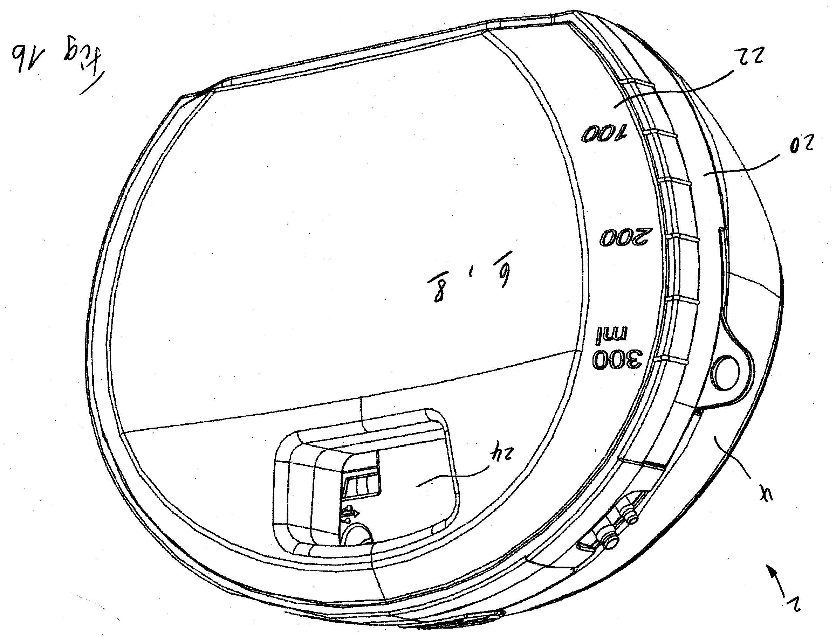

Figuren 1a, b zeigt eine insgesamt mit dem Bezugszeichen 2 bezeichnete Vorrichtung zur Bereitstellung von Unterdruck für medizinische Anwendungen. Die Vorrichtung 2 ist am Körper eines Benutzers tragbar (mobiler Betrieb), sie ist aber auch stationär betreibbar (stationärer Betrieb). Im mobilen Betrieb könnte die Vorrichtung über einen Gürtel, Tragriemen oder dergleichen mitgeführt und am Körper getragen werden, wobei hierfür rein beispielhaft eine Befestigungsöse 3 dargestellt ist. Im stationären Betrieb ist die Vorrichtung auf eine vorzugsweise ebene Unterlage stellbar. Die Vorrichtung 2 umfasst einen ersten Gehäuseteil 4, in dem eine Unterdruck erzeugende Einrichtung sowie elektrische und elektronische Steuerkomponenten für die Vorrichtung (2) insgesamt aufgenommen sind, einschließlich Batterien oder vorzugsweise wiederaufladbare Akkus. Des Weiteren umfasst die Vorrichtung 2 einen zweiten Gehäuseteil 6, welcher von einem Behälter 8 zur Aufnahme von Körperflüssigkeiten in seinem Inneren, insbesondere zur Aufnahme von aus einer Wunde abgesaugten Wundsekreten, ausgebildet ist. Der Behälter 8 ist als wegwerfbarer Einmalartikel ausgebildet. Der Behälter 8 umfasst oben Anschlussstutzen 10, 12 für eine nicht dargestellte Saugleitung und eine Leitung zur Medienzuführung in Richtung auf den Körper des Patienten oder zur Bildung eines Messkanals. Die nicht dargestellte Saugleitung führt dann im beispielhaften Betrieb der Vorrichtung zur Unterdrucktherapie von Wunden zu einem die Wunde druckdicht verschließenden Wundverband und kommuniziert dort, beispielsweise über einen Port, mit einem Wundraum, um in dem Wundraum Unterdruck anzulegen, aufrechtzuerhalten oder zu variieren und Wundsekrete in den Behälter 8 abzusaugen. Hierfür kommuniziert der Behälter 8 mit der Unterdruck erzeugenden Einrichtung im ersten Gehäuseteil 4.

Der Anschlussstutzen 12 führt ebenfalls über eine nicht dargestellte Leitung, die einen Mess- oder Spülkanal bildet, in Richtung zum Körper des Benutzers, insbesondere zu einer Wunde. Auf noch näher zu beschreibende Weise kommuniziert der Anschlussstutzen 12 durch das Innere des Behälters 8 hindurch mit dem ersten Gehäuseteil 4 und ist von dort mit einem Zuführmedium, insbesondere Spülmedium, beaufschlagbar oder steht in Verbindung mit einem Messanschluss.

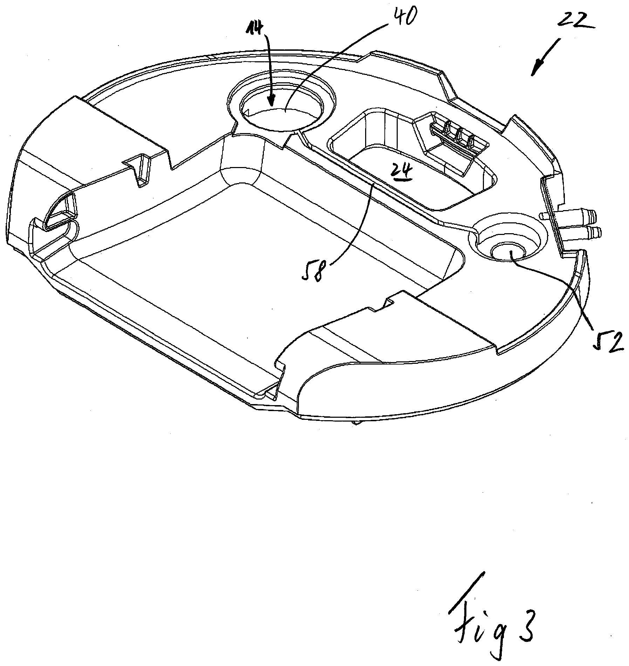

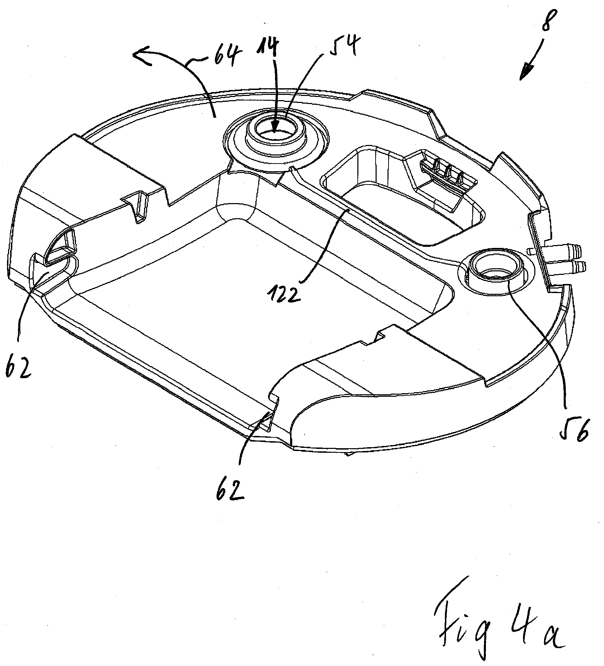

Der Anschlussstutzen 10 für die Saugleitung mündet im Inneren des Behälters 8. Das Innere des Behälters 8 kommuniziert über eine noch näher zu beschreibende Unterdruckschnittstelle 14 (Figur 3, 4a) mit der Unterdruck erzeugenden Einrichtung innerhalb des ersten Gehäuseteils 4, wenn der Behälter 8 in der in Figur 1 gezeigten Betriebsposition an dem ersten Gehäuseteil 4 fixiert ist. Hierfür ist der Behälter 8 manuell an dem ersten Gehäuseteil 4 positionierbar und dann manuell in eine mechanisch fixierte und mechanisch wieder lösbare Montageposition, insbesondere Verrastung, bringbar. In dieser Montageposition wird dann automatisch eine Unterdruckkommunikation zwischen dem Inneren des Behälters 8 und der Unterdruck erzeugenden Einrichtung einerseits sowie eine Strömungskommunikation zwischen dem Anschlussstutzen 12 und dem zugeordneten Mess- oder Spülanschluss bei dem ersten Gehäuseteil 4 realisiert, so wie dies grundsätzlich in DE 10 2009 038 130.9 beschrieben ist, so dass hierauf für Offenbarungszwecke Bezug genommen wird.

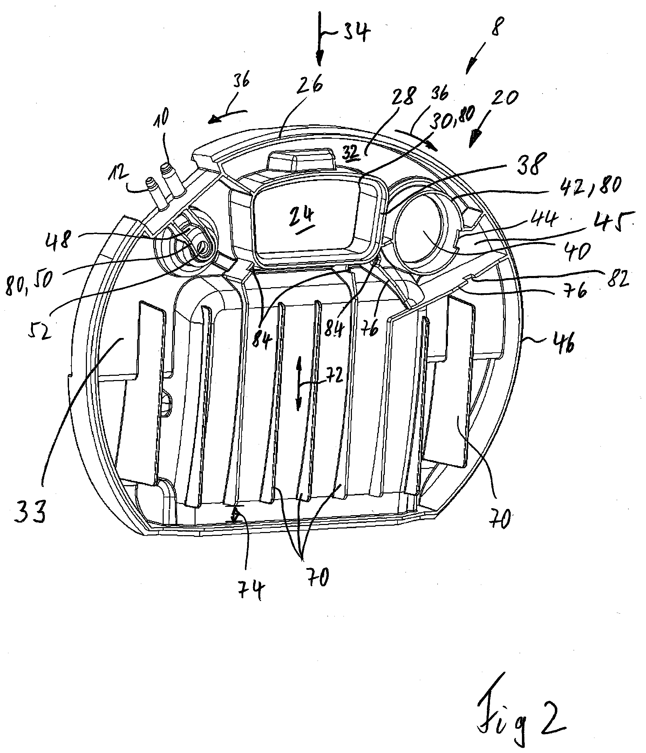

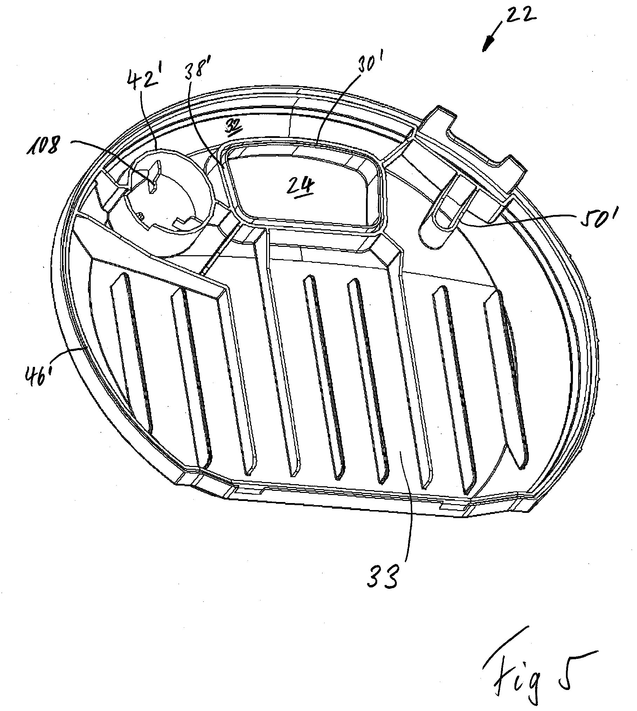

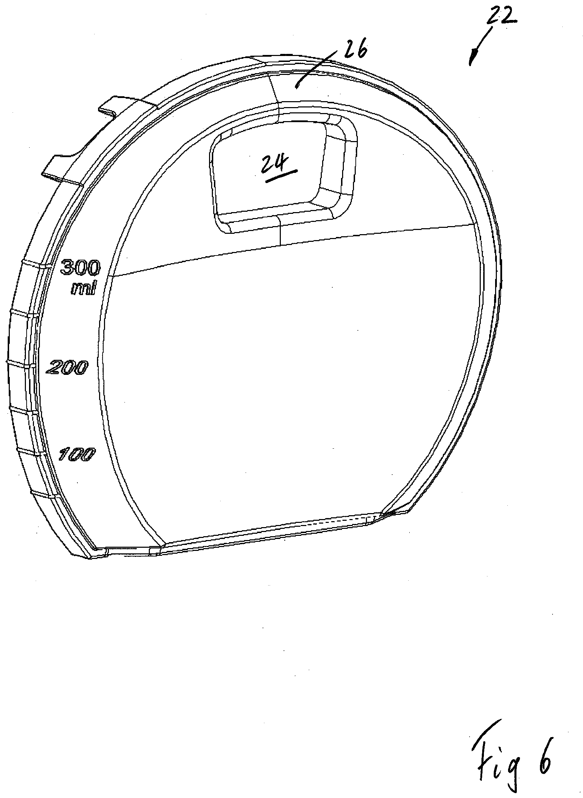

Die Figuren 2 bis 6 zeigen die Ausbildung des den zweiten Gehäuseteil 6 bildenden Behälters 8. Der Behälter 8 umfasst ein erstes dem ersten Gehäuseteil 4 zugewandtes Halbschalenteil 20 und ein zweites von dem ersten Gehäuseteil 4 abgewandtes im mobilen Betrieb an den Körper des Benutzers anlegbares zweites Halbschalenteil 22 (Figuren 5, 6), die zusammengesetzt das Innere des Behälters (8) begrenzen. Der Behälter 8 umfasst des Weiteren eine Eingriffsöffnung 24, die im beispielhaft dargestellten Fall als durch den Behälter 8 hindurchgehende Durchgriffsöffnung ausgebildet ist und von beiden Halbschalenteilen 20, 22 gebildet bzw. begrenzt wird. Die Eingriffsöffnung 24 ist in dem ersten Behälter 8 ungefähr in einem oberen Viertel und mittig ausgebildet, so dass sich ein Schwerpunkt des Behälters und der Vorrichtung insgesamt ungefähr unterhalb der Eingriffsöffnung 24 befindet. Der Behälter 8 und auch die gesamte Vorrichtung sind durch manuellen Eingriff in die Eingriffsöffnung 24 mit den Fingern eines Benutzers ergreifbar. Dabei umgreift der Benutzer mit der Hand einen oberhalb der Eingriffsöffnung 24 ausgebildeten verhältnismäßig breiten Steg 26, der dabei einen Griffbereich bildet. Beim Umgreifen dieses Stegs 26 kann der Benutzer, vorzugsweise mit dem Daumen, ein Entriegelungsorgan 27 (Figur 1a) bedienen, so dass der Behälter aus seiner in Figur 1a dargestellten Betriebsposition an dem ersten Gehäuseteil 4 gelöst und nach schräg oben abgehoben werden kann. Auch dies ist in DE 2009 038 130.9 beschrieben, so dass hierauf für Offenbarungszwecke Bezug genommen wird.

Man erkennt aus den Figuren, dass sich das Innere des Behälters 8 bis in einen Bereich 28 angrenzend an eine die Eingriffsöffnung 24 begrenzende Wandung 30 erstreckt. In dem beispielhaft und bevorzugt dargestellten Fall ist dieser Bereich 28 oberhalb einer die Eingriffsöffnung 24 nach oben begrenzenden Wandung 30 ausgebildet. Dieser Bereich 28 ist von einer Kammer 32 begrenzt oder gebildet, die derart abgeschlossen, d.h. abgeschottet, ausgebildet ist, dass sie nicht mit anderen angrenzenden Kammern oder Bereichen im Inneren des Behälters 8 kommuniziert. Dies bedeutet, dass in eine Flüssigkeitssammelkammer 33 im Inneren des Behälters 8 angesaugte Körperflüssigkeit, insbesondere aus einer Wunde abgesaugtes Wundsekret, auch beim Verkippen oder kurzzeitigen Neigen des Behälters 8 nicht in diese Kammer 32 eindringen kann. Somit bildet die Kammer 32 von außerhalb des Behälters 8 betrachtet eine Art Sichtschutz im Bereich der Eingriffsöffnung 24 und im Bereich des umgreifbaren Stegs 26. Im beispielhaft und bevorzugt dargestellten Fall überfängt diese Kammer 32 in vertikaler Richtung 34 von oben betrachtet die Eingriffsöffnung 24 vollständig. Die Kammer 32 erstreckt sich auch in Richtung der Pfeile 36 zu zwei einander gegenüberliegenden Seiten hin über die Eingriffsöffnung 24 hinweg. Man erkennt aus Figur 2 ferner, dass sich die Kammer 32 auch angrenzend an eine die Eingriffsöffnung 24 seitlich begrenzende Wandung 38 erstreckt. Sie wird dort nach außen hin von einer Wandung 42 begrenzt, die eine Kammer 40 zur Aufnahme eines Filters bildet.

Im Unterschied zur Kammer 32 ist die den Filter aufnehmende Kammer 40 strömungsmäßig nicht abgeschlossen. Sie kommuniziert zum einen mit der Unterdruck erzeugenden Einrichtung in dem ersten Gehäuseteil 4 und zum anderen über eine Unterdruck kommunizierende Öffnung 44 in der Wandung 42 mit einer Vorkammer 45. Das zweite Halbschalenteil 22 ist bezüglich der Wandungen senkrecht zur Teilungsebene komplementär zu dem ersten Halbschalenteil 20 ausgebildet. Man sieht in Figur 5 eine komplementär zur Wandung 42 ausgebildete Wandung 42'. Die die Eingriffsöffnung 24 begrenzende und in einer Umfangsrichtung durchgehend erstreckte Wandung 30, 38 sowie eine äußere Wandung 46 und die zugehörigen komplementären Wandungen 30', 38', 46' sind in der Trennebene in ihrem Stoßbereich so ausgebildet, dass dort wenigstens eine Abstufung der Stoßenden ausgebildet ist, was einerseits eine selbstzentrierende Wirkung hat und andererseits eine leichtere Abdichtbarkeit und Verklebung der Halbschalenteile 20, 22 ermöglicht.

Ausgehend von der Unterdruck erzeugenden Einrichtung kann durch den in Figuren 2-6 nicht dargestellten Filter hindurch und durch die Öffnung 44 in der Wandung 42 ein Unterdruck im Inneren des Behälters 8 aufgebaut werden. Da der Anschlussstutzen 10 direkt in das Innere des Behälters 8 mündet, kann über die nicht dargestellte Saugleitung einerseits ein Unterdruck an eine Wunde angelegt und andererseits Körperflüssigkeit von dort in das Innere des Behälters 8 angesaugt werden.

Der weitere Anschlussstutzen 12 mündet von außen betrachtet in eine weitere zum übrigen Innenraum des Behälters 8 abgeschlossene Kammer 48, die von in Umfangsrichtung geschlossenen Wandungen 50 und entsprechend 50' der beiden Halbschalenteile 20, 22 begrenzt wird. Von dieser Kammer 48 führt in Tiefenrichtung, also senkrecht zu der Trennebene, ein Durchgangskanal 52 in Richtung auf den ersten Gehäuseteil 4, also wieder nach außerhalb des Behälters 8.

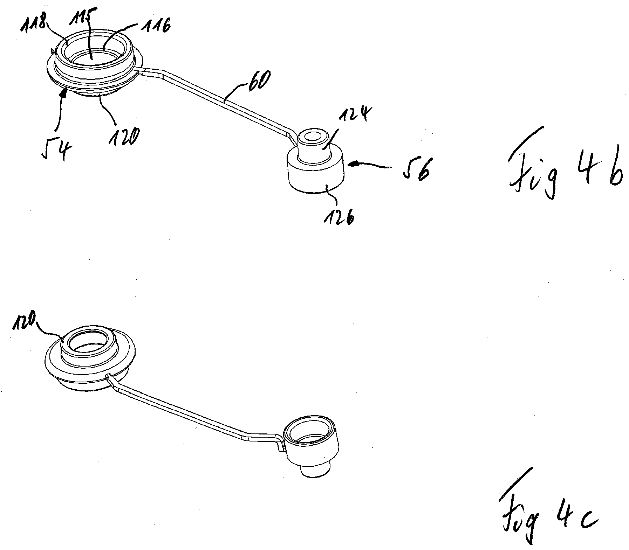

Die Figuren 3 und 4 zeigen das erste Halbschalenteil 20 von außen, also mit Blick auf die dem ersten Gehäuseteil 4 in der Betriebsposition zugewandte Seite. Figuren 3 und 4a zeigen, dass an das erste Halbschalenteil 20 von außen zur Abdichtung der Kammer 40 für den Filter und zur Abdichtung des Durchgangskanals 52 ein Dichtelement 54 bzw. 56 vorzugsweise direkt angespritzt werden kann. Bei dem schmalen Kanal 58 handelt es sich um eine Zuführstrecke von Spritzgussmaterial, wodurch ein die beiden Dichtelemente 54, 56 verbindender Steg 60 gebildet wird. Man erkennt aus Figur 4a, dass die Dichtelemente 54, 56 einen nach außen vorstehenden konzentrischen, zylindrischen Abschnitt aufweisen. Beim Ansetzen des Behälters 8 an den ersten Gehäuseteil 4 wird der Behälter 8 mit zwei nach unten ungefähr U-förmig öffnenden Lagerbereichen 62 schräg von oben auf einen komplementär hierzu ausgebildeten Gegenlagerbereich bei dem ersten Gehäuseteil 4 aufgesetzt und dann in Richtung des Pfeils 64, also ungefähr quer zu der generellen Scheibenform des Behälters 8, gegen den ersten Gehäuseteil 4 gedrückt, wobei es dann zu der eingangs erwähnten Verriegelung kommt. Hierbei werden die zylindrischen Abschnitte der Dichtungselemente 54, 56 gegen von dem ersten Gehäuseteil 4 vorstehende vorzugsweise konusförmige Anschlussstücke gedrückt, wodurch eine dichtende Strömungsverbindung geschaffen wird.

Das Innere des Behälters 8 ist durch eine Vielzahl von Rippen partioniert (Figur 2). Rippen 70 eines ersten Typs erstrecken sich im Wesentlichen in vertikaler Richtung 72, also in Richtung des Pfeils 34, innerhalb der Flüssigkeitssammelkammer 33. Sie enden unten in einem Abstand 74 von vorzugsweise wenigstens 5 mm, damit sich die Flüssigkeit bei Neigung des Behälters 8 und auch bei regulärem Ansaugen der Flüssigkeit durch den Anschlussstutzen 10 gleichmäßig im Inneren des Behälters verteilen kann.

Des Weiteren gibt es Rippen 76 eines zweiten schräg verlaufenden Typs. Mit Hilfe dieser Rippen wird ein Schwappen der in dem Behälter 8 aufgenommenen Flüssigkeit in Richtung auf die Filterkammer 40 verhindert. Eine solche Rippe 76 begrenzt die schon erwähnte Vorkammer 45 zur Filterkammer 40.

Zur Unterdruckkommunikation ist in der schräg verlaufenden Rippe 76 eine verhältnismäßig klein ausgebildete Durchgangsöffnung 82 ausgebildet, welche eine Unterdruckkommunikation zwischen der Vorkammer 45 und dem übrigen Innenraum des Behälters 8 herbeiführt und dabei aber einen großen Widerstand für eindringende Flüssigkeit darstellt, welche den Filter zusetzen könnte.

Ferner gibt es Rippen 80 eines dritten Typs, die in Umfangsrichtung aufeinander zurückgeführt sind. Sie bilden z.B. die Wandung 42 für die Kammer 40 oder die Wandung 50 für die Kammer 48. Auch die in Umfangsrichtung auf sich selbst zurückgeführte Wandung 30, die die Eingriffsöffnung 24 begrenzt, kann als Rippe 80 des dritten Typs bezeichnet werden.

Auch bei denjenigen Rippen 70, 76, die nach oben bis zu einer schräg oder horizontal verlaufenden Wandung erstreckt sind, ist vorzugsweise ganz oben eine Durchgangsöffnung 84 zum Druckausgleich und Gasdurchtritt vorgesehen, so dass keine Luft durch die Flüssigkeit hindurch geführt werden muss.

Die beiden Halbschalenteile 20, 22 werden vorzugsweise durch Kleben oder Thermoschweißen unlösbar und dichtend gegeneinander gefügt und bilden so den das zweite Gehäuseteil 6 der Vorrichtung 2 bildenden Behälter 8. Durch die gegen die Flüssigkeit und den übrigen Innenraum des Behälters 8 abgeschlossene Kammer 32 wird der manuell ergreifbare Steg oder Griffbereich 26 vor Kontamination durch im Inneren des Behälters 8 aufgenommene Körperflüssigkeit geschützt; außerdem wird hierdurch ein Sichtschutz gebildet, der von oben betrachtet den Bereich der Eingriffsöffnung 24 teilweise oder vorzugsweise vollständig überfängt.

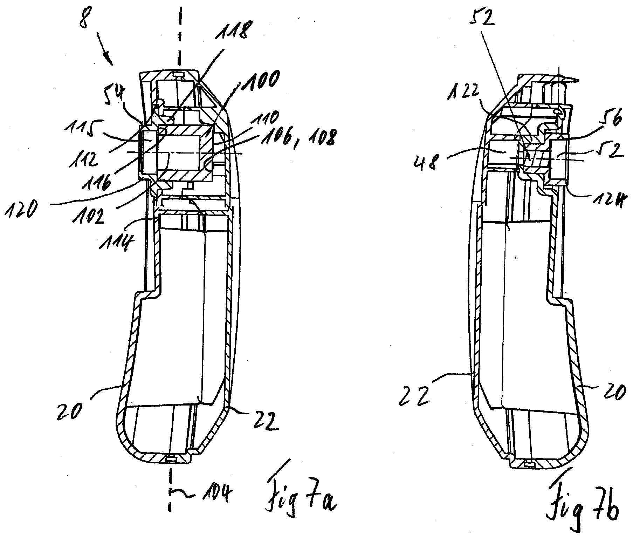

Figur 7a zeigt die Anordnung des Filters 100 in dem Behälter 8. Man erkennt, dass der Filter 100 in einer axialen Richtung 102, die auch der Fügerichtung der Halbschalenteile 20, 22 entspricht und senkrecht zu einer Teilungsebene 104 zwischen den Halbschalenteilen 20, 22 verläuft, zwischen dem Dichtungselement 54 und einem Innenbereich 106 des Halbschalenteils 22 angeordnet ist. Dieser Innenbereich 106 ist bevorzugtermaßen gebildet von vorzugsweise mehreren Rippen 108, die auch aus Figur 5 besonders gut ersichtlich sind. Sie erstrecken sich vorteilhafterweise auch in axialer Richtung 102 und vorzugsweise zusätzlich radial zu der axialen Richtung 102. Der Filter 100 weist beispielhaft eine topfförmige Gestalt auf, wobei er sich mit der Außenseite des Topfbodens 110 axial gegen die Rippen 108 abstützt. Das Dichtungselement 54 ist aus einem elastisch nachgiebigen Material ausgebildet. Es ist in einer Durchgangsöffnung 112 in einer Wandung 114 des ersten Halbschalenteils 20 des Behälters 8 angeordnet, und zwar vorzugsweise dadurch, dass es dort direkt angespritzt ist. Das Dichtungselement 54 ist vorzugsweise konzentrisch ausgebildet und umfasst eine Durchgangsöffnung 115 und darin eine Abstufung 116, gegen die der Filter 100 in axialer Richtung 102 anliegt. Hierbei wird das Dichtungselement 54 geringfügig deformiert, wodurch eine in axialer Richtung 102 wirkende Einspannkraft resultiert, die den Filter 100 in seiner bestimmungsgemäßen Montageposition hält. Wie aus Figur 7a und Figur 4b ersichtlich, umfasst das Dichtungselement 54 einen in das Innere des Behälters 8 vorstehenden rohrförmigen Ansatz 118, in den der Filter 100 axial eingreift. Der Innendurchmesser des rohrförmigen Ansatzes 118 ist dabei derart auf den Außenumfang des Filters 100 abgestimmt, dass dieser darin klemm- und reibschlüssig fixiert ist, was das Zusammenfügen der Halbschalenteile 20, 24 erleichtert.

Des Weiteren umfasst das Dichtungselement 54 einen von dem Behälter 8 in Richtung auf das erste Gehäuseteil 4 vorstehenden rohrförmigen Ansatz 120, mittels dessen eine Strömungsverbindung zu der Unterdruck erzeugenden Einrichtung in dem ersten Gehäuseteil 4 herstellbar ist. Beispielsweise kann der rohrförmige Ansatz 120 gegen einen beispielsweise konusförmigen Ansatz automatisch dichtend und eine Strömungsverbindung bildend angelegt werden, wenn der Behälter 8 in seine bestimmungsgemäße Montageposition an dem ersten Gehäuseteil 4 gebracht wird.

Figur 7b zeigt eine Figur 7a entsprechende Schnittansicht jedoch mit entgegengesetzter Blickrichtung durch das weitere Dichtungselement 56, das wie in Figuren 4b, c dargestellt ausgebildet ist. Es weist wie das Dichtungelement 54 einen rohrförmigen Ansätze 122, 124 auf. Sein Durchgangskanal 52 mündet in der oben erwähnten Kammer 48, die gegenüber dem übrigen Innenraum des Behälters 8 abgedichtet ist und über den Anschlussstutzen 12 (Figur 2) mit einer nicht dargestellten Spül- oder Messleitung kommuniziert.

Beide Dichtungselemente 54, 56 sind vorzugsweise in demselben Spritzgießvorgang hergestellt und direkt an das erste Halbschalenteil 20 angespritzt. Um in einer hierfür zu verwendenden Spritzgussform nur einen Anschnitt vorsehen zu müssen, sind die beiden Dichtungselemente 54, 56 über eine gemeinsame Füllstrecke 126 verbunden, die demzufolge mit elastomerem Material befüllt ist (s. Figuren 4).

## Patentansprüche

1. Flüssigkeitsaufnahmebehälter (8) für eine am Körper eines Benutzers tragbare und zusätzlich stationär betreibbare Vorrichtung (2) zur Bereitstellung von Unterdruck für medizinische Anwendungen, insbesondere zur Unterdruckbehandlung von Wunden am menschlichen oder tierischen Körper, wobei die Vorrichtung (2) einen ersten Gehäuseteil (4) mit einer Unterdruck erzeugenden Einrichtung und den nach Gebrauch wegwerfbaren Behälter (8) zur Aufnahme von Körperflüssigkeiten in seinem Inneren, insbesondere von aus einer Wunde abgesaugten Wundsekreten, umfasst, wobei der Behälter (8) einen zweiten Gehäuseteil (6) der Vorrichtung (2) bildet und an dem ersten Gehäuseteil (4) der Vorrichtung (2) manuell befestigbar und manuell lösbar ist und im befestigten Zustand das Innere des Behälters (8) von der Unterdruck erzeugenden Einrichtung mit Unterdruck beaufschlagbar ist, und wobei am Behälter (8) ein Anschluss (10) für eine zum Körper führende Saugleitung vorgesehen ist, so dass eine Unterdruckkommunikation zwischen der Unterdruck erzeugenden Einrichtung, dem Behälter (8) und der zum Körper führenden Saugleitung herstellbar ist, wobei der Behälter (8) zwei Halbschalenteile (20, 22) umfasst, die gegeneinander unlösbar gefügt sind und dabei das Innere des Behälters begrenzen, wobei auch ein Bereich zur Aufnahme eines Filters (100) im Inneren des Behälters (8) gebildet wird, **dadurch gekennzeichnet, dass** der Filter (100) in einer axialen Richtung (102), die ungefähr senkrecht zu einer Teilungsebene (104) der Halbschalenteile (20, 22) verläuft, einerseits gegen einen Innenbereich (106) des vom ersten Gehäuseteil (4) abgewandten Halbschalenteils (22) und andererseits gegen ein Dichtungselement (54) unter axialer Spannung anliegt, welches Dichtungselement (54) an das dem ersten Gehäuseteil (4) zugewandte Halbschalenteil (20) angefügt ist, wobei die axiale Spannung durch Zusammenfügen der Halbschalenteile (20, 22) und damit einhergehender Verformung des Dichtungselements (54) durch den Filter (100) gebildet ist.

2. Behälter nach Anspruch 1, **dadurch gekennzeichnet, dass** der Filter (100) gegen Rippen (108) anliegt, die im Innenbereich (106) des Halbschalenteils (22) ausgebildet sind.

3. Behälter nach Anspruch 1 oder 2, **dadurch gekennzeichnet, dass** der Filter (100) in einer Kammer (40) aufgenommen ist, die von einer auf sich selbst zurückgeführten Wandung (42) begrenzt ist.

4. Behälter nach Anspruch 1, 2 oder 3, **dadurch gekennzeichnet, dass** der Filter (100) zylindrisch, topf- oder konusförmig ausgebildet ist.

5. Behälter nach einem oder mehreren der vorstehenden Ansprüche, **dadurch gekennzeichnet, dass** das Dichtungselement (54) ein Spritzgussteil aus einem elastisch nachgiebigen Material ist, welches in einer Durchgangsöffnung (112) in einer Wandung (114) des ersten Halbschalenteils (20) des Behälters (8) vorzugsweise formschlüssig gehalten ist.

6. Behälter nach einem oder mehreren der vorstehenden Ansprüche, **dadurch gekennzeichnet, dass** das Dichtungselement (54) ein Spritzgussteil aus einem elastisch nachgiebigen Material ist, welches in einer Durchgangsöffnung (112) in einer Wandung (114) des ersten Halbschalenteils (20) des Behälters (8) angeordnet und an die Wandung (114) angespritzt ist.

7. Behälter nach einem oder mehreren der vorstehenden Ansprüche, **dadurch gekennzeichnet, dass** das Dichtungselement (54) eine axiale Durchgangsöffnung (115) aufweist.

8. Behälter nach einem oder mehreren der vorstehenden Ansprüche, **dadurch gekennzeichnet, dass** das Dichtungselement (54) bezüglich der axialen Richtung (102) konzentrisch ausgebildet ist.

9. Behälter nach einem oder mehreren der vorstehenden Ansprüche, **dadurch gekennzeichnet, dass** das Dichtungselement (54) eine Abstufung (116) bezüglich der axialen Richtung (102) aufweist, gegen die der Filter (100) anliegt und die vorzugsweise radial innen ausgebildet ist.

10. Behälter nach einem oder mehreren der vorstehenden Ansprüche, **dadurch gekennzeichnet, dass** das Dichtungselement (54) einen in das Innere des Behälters (8) vorstehenden rohrförmigen Ansatz (118) aufweist, in den der Filter (100) eingreift.

11. Behälter nach einem oder mehreren der vorstehenden Ansprüche, **dadurch gekennzeichnet, dass** das Dichtungselement (54) einen von dem Behälter (8) in Richtung auf das erste Gehäuseteil (4) vorstehenden rohrförmigen Ansatz (120) aufweist, mittels dessen eine Strömungsverbindung zu der Unterdruck erzeugenden Einrichtung herstellbar ist.

12. Behälter nach einem oder mehreren der vorstehenden Ansprüche, **dadurch gekennzeichnet, dass** ein weiteres Dichtungselement (56) vorgesehen ist, welches zwischen einer Kammer (48) im Inneren des Behälters (8) und einem Medien- oder Messanschluss in dem ersten Gehäuseteil (4) angeordnet ist und von dem Behälter (8) in Richtung auf das erste Gehäuseteil (4) mit einem rohrförmigen Ansatz (124) vorsteht.

13. Behälter nach Anspruch 12, **dadurch gekennzeichnet, dass** das weitere Dichtungselement (56) ein Spritzgussteil aus einem elastisch nachgiebigen Material ist, welches vorzugsweise in einer Durchgangsöffnung in einer Wandung des ersten Halbschalenteils (20) des Behälters (8) angeordnet und an die Wandung angespritzt ist.

14. Behälter nach Anspruch 12 oder 13, **dadurch gekennzeichnet, dass** die Dichtungselemente (54, 56) im selben Spritzgießvorgang ausgebildet sind und bei dem Behälter (8) über eine mit elastomerem Material gefüllte Füllstrecke (126) verbunden sind.

15. Verfahren zur Herstellung des Behälters nach einem oder mehreren der vorstehenden Ansprüche, die folgenden Verfahrensschritte umfassend:
- Bereitstellen der zwei Halbschalenteile (20, 22),
- Anspritzen des Dichtungselements (54) an das dem ersten Gehäuseteil (4) zugewandte Halbschalenteil (20),
- Anordnen des Filters (100) an dem Dichtungselement (54) oder Einsetzen des Filters (100) in das Dichtungselement (54),
- Zusammenfügen der zwei Halbschalenteile (20, 22) unter Zwischenordnung des Filters (100), der dabei in einer axialen Richtung (102) gegen das Dichtungselement (54) gedrückt wird, und Erzeugen einer Spannung in der axialen Richtung (102) durch das Zusammenfügen der Halbschalenteile (20, 22) und damit einhergehender Verformung des Dichtungselements (54) durch den dagegen gedrückten Filter (100) und
- unlösbares Verbinden der zwei Halbschalenteile (20, 22) miteinander.

16. Am Körper eines Benutzers tragbare und zusätzlich stationär betreibbare Vorrichtung (2) zur Bereitstellung von Unterdruck für medizinische Anwendungen, insbesondere zur Unterdruckbehandlung von Wunden am menschlichen oder tierischen Körper, mit einer Unterdruck erzeugenden Einrichtung und einem nach Gebrauch wegwerfbaren Behälter (8) zur Aufnahme von Körperflüssigkeiten in seinem Inneren, insbesondere von aus einer Wunde abgesaugten Wundsekreten, wobei die Unterdruck erzeugende Einrichtung in oder an einem ersten Gehäuseteil (4) der Vorrichtung angeordnet ist und der Behälter (8) einen zweiten Gehäuseteil (6) der Vorrichtung bildet und an dem ersten Gehäuseteil (4) der Vorrichtung manuell befestigbar und manuell lösbar ist und im befestigten Zustand das Innere des Behälters (8) von der Unterdruck erzeugenden Einrichtung mit Unterdruck beaufschlagbar ist, und wobei am Behälter (8) ein Anschluss (10) für eine zum Körper führende Saugleitung vorgesehen ist, so dass eine Unterdruckkommunikation zwischen der Unterdruck erzeugenden Einrichtung, dem Behälter (8) und der zum Körper führenden Saugleitung herstellbar ist, gekennzeichnetdurch einen Behälter (8) nach einem oder mehreren der vorstehenden Ansprüche 1-14.

## Claims

1. Liquid-accommodating container (8) for a device (2), which can be worn on the body of a user and additionally operated in a stationary manner, for providing vacuum for medical applications, in particular for the vacuum treatment of wounds on a human or animal body, wherein the device (2) comprises a first housing part (4) having a vacuum-generating apparatus and the container (8), which can be discarded after use, for collecting bodily fluids in the interior of the container, in particular wound secretions suctioned out of a wound, wherein the container (8) forms a second housing part (6) of the device (2), and is manually fastenable to and manually detachable from the first housing part (4) of the device (2) and, when in the fastened state, a vacuum can be applied to the interior of the container (8) by the vacuum-generating apparatus, and wherein a connection (10) for a suction line leading to the body is provided on the container (8), such that vacuum communication between the vacuum-generating apparatus, the container (8), and the suction line leading to the body can be established, wherein the container (8) comprises two half-shell parts (20, 22), which are unseparably joined to each other and bound the interior of the container in the joined state, wherein a region for accommodating a filter (100) is also formed in the interior of the container (8), **characterized in that,** the filter (100) lies against an inner region (106) of the half-shell part (22) facing away from the first housing part (4) on one side and against a sealing element (54) on the other side under axial tension in an axial direction (102) extending approximately perpendicularly to a dividing plane (104) of the half-shell parts (20, 22), which sealing element (54) is attached to the half-shell part (20) facing the first housing part (4), wherein the axial tension is formed by the joining of the half-shell parts (20, 22) and associated deformation of the sealing element (54) by the filter (100).

2. Container according to the claim 1, **characterized in that** the filter (100) rests against ribs (108), which are formed in the interior (106) of the half-shell part (22).

3. Container according to either one of the claims 1 or 2, **characterized in that** the filter (100) is accommodated in a chamber (40) that is bounded by a wall (42) that joins onto itself.

4. Container according to any one of the claims 1, 2 or 3, **characterized in that** the filter (100) is constituted cylindrically, pot shaped or conically.

5. Container according to one or more of the previous claims, **characterized in that** the sealing element (54) is an injection molded part made of elastically compliant material, which is retained, preferably by a positive form locking connection, in a through opening (112) in a wall (114) of the first half-shell part (20) of the container (8).

6. Container according to one or more of the previous claims, **characterized in that** the sealing element (54) is an injection molded part made of elastically compliant material, which is disposed in a through opening (112) in a wall (114) of the first half-shell part (20) of the container (8) and injection molded onto the wall (114).

7. Container according to one or more of the previous claims, **characterized in that** the sealing element (54) has an axial through opening (115).

8. Container according to one or more of the previous claims, **characterized in that** the sealing element (54) is constituted concentrically with respect to the axial direction (102).

9. Container according to one or more of the previous claims, **characterized in that** the sealing element (54) comprises a step (116) with respect to the axial direction (102) against which the filter (100) lies and which is preferably constituted radially inward.

10. Container according to one or more of the previous claims, **characterized in that** the sealing element (54) comprises a tubular projection (118) that projects into the interior of the container (8), in which the filter (100) engages.

11. Container according to one or more of the previous claims, **characterized in that** the sealing element (54) comprises a tubular projection (120) that projects from the container (8) toward the first housing part (4), by means of which a fluid-dynamic connection can be created to the vacuum-generating apparatus.

12. Container according to one or more of the previous claims, **characterized in that** a further sealing element (56) is provided, which is disposed between a chamber (48) in the interior of the container (8) and a medium or measuring connection in the first housing part (4) and which projects from the container (8) toward the first housing part with a tubular projection (124).

13. Container according to the claim 12, **characterized in that** the further sealing element (56) is an injection-molded part made of elastically compliant material, which is preferably disposed in an opening in a wall of the first half-shell part (20) of the container (8) and injection molded onto the wall.

14. Container according to either one of the claims 12 or 13, **characterized in that** the sealing elements (54, 56) are formed in the same injection-molding process and are connected at the container (8) via a filling gate (126) filled with elastomeric material.

15. Method for the manufacture of the container according to one or more of the previous claims, comprising the following process steps:
- Provision of the two half-shell parts (20, 22),
- Injection molding of the sealing element (54) onto the half-shell part (20) facing the first housing part (4),
- Disposition of the filter (100) on the sealing element (54) or insertion of the filter (100) into the sealing element (54),
- Joining of the two half-shell parts (20, 22) with interposition of the filter (100), which is pressed in an axial direction (102) against the sealing element (54), and creation of a stress in the axial direction (102) by joining the half-shell parts (20, 22) and associated deformation of the sealing element (54) by the filter (100) being pressed against it, and
- Non-detachable joining of the two half-shell parts (20, 22) to each other.

16. Device (2), which can be worn on the body of a user and additionally can be operated in a stationary manner, for providing a vacuum for medical applications, in particular for the vacuum treatment of wounds on a human or animal body, having a vacuum-generating apparatus and a container (8), which can be discarded after use, for accommodating bodily fluids in the interior of the container, in particular wound secretions suctioned from a wound, wherein the vacuum-generating apparatus is disposed in or at a first housing part (4) of the device and the container (8) forms a second housing part (6) of the device, and is manually fastenable to and manually detachable from the first housing part (4) of the device and, when in the fastened state, a vacuum can be applied to the interior of the container (8) from the vacuum generating apparatus, and wherein a connection (10) for a suction line leading to the body is provided on the container (8), such that vacuum communication between the vacuum-generating apparatus, the container (8), and the suction line leading to the body can be established, **characterized by** a container (8) according to one or more of the previous claims 1-14.

## Revendications

1. Récipient récepteur de liquide (8) pour un dispositif (2) qui peut être porté sur le corps d'un utilisateur et peut, en sus, fonctionner de manière stationnaire et qui est destiné à fournir de la pression négative pour des applications médicales, en particulier pour le traitement par pression négative de plaies sur le corps humain ou animal, dans lequel ledit dispositif (2) comprend une première partie de boîtier (4) comprenant un dispositif générant de la pression négative et le récipient (8) jetable après l'utilisation et destiné à recevoir dans son intérieur des liquides corporels, en particulier des exsudats aspirés d'une plaie, dans lequel ledit récipient (8) forme une deuxième partie de boîtier (6) du dispositif (2) et peut être fixé manuellement sur la première partie de boîtier (4) du dispositif (2) et en être détaché manuellement, et, lorsqu'il est fixé, l'intérieur du récipient (8) peut être mis sous pression négative par ledit dispositif générant de la pression négative, et dans lequel un raccord (10) pour un conduit d'aspiration menant vers le corps est prévu sur le récipient (8) de sorte qu'une communication par pression négative peut être établie entre le dispositif générant de la pression négative, le récipient (8) et ledit conduit d'aspiration menant vers le corps, dans lequel le récipient (8) comprend deux parties en demi-cuvette (20, 22) qui sont jointes l'une contre l'autre de manière inamovible tout en délimitant l'intérieur du récipient, dans lequel une zone de réception d'un filtre (100) est également formée à l'intérieur du récipient (8), **caractérisé par le fait que** ledit filtre (100) s'applique sous tension axiale, dans une direction axiale (102) qui s'étend à peu près perpendiculairement à un plan de séparation (104) des parties en demi-cuvette (20, 22), d'une part contre une zone intérieure (106) de la partie en demi-cuvette (22) montrant dans la direction opposée à la première partie de boîtier (4) et d'autre part contre un élément d'étanchéité (54), lequel élément d'étanchéité (54) est joint à la partie en demi-cuvette (20) montrant en direction de la première partie de boîtier (4), la tension axiale étant réalisée par l'assemblage des parties en demi-cuvette (20, 22) et la déformation de l'élément d'étanchéité (54) par le filtre (10), laquelle va de pair avec celui-ci.

2. Récipient selon la revendication 1, **caractérisé par le fait que** le filtre (100) s'applique contre des nervures (108) qui sont réalisées dans la zone intérieure (106) de la partie en demi-cuvette (22).

3. Récipient selon la revendication 1 ou 2, **caractérisé par le fait que** le filtre (100) est reçu dans une chambre (40) qui est délimitée par une paroi (42) ramenée sur elle-même.

4. Récipient selon la revendication 1, 2 ou 3, **caractérisé par le fait que** le filtre (100) est réalisé de manière à être cylindrique, en forme de pot ou de cône.

5. Récipient selon l'une ou plusieurs des revendications précédentes, **caractérisé par le fait que** ledit élément d'étanchéité (54) est une pièce moulée par injection en un matériau élastiquement flexible qui est maintenue, de préférence à engagement positif, dans une ouverture de passage (112) dans une paroi (114) de la première partie en demi-cuvette (20) du récipient (8).

6. Récipient selon l'une ou plusieurs des revendications précédentes, **caractérisé par le fait que** ledit élément d'étanchéité (54) est une pièce moulée par injection en un matériau élastiquement flexible qui est disposée dans une ouverture de passage (112) dans une paroi (114) de la première partie en demi-cuvette (20) du récipient (8) et est surmoulée par injection sur ladite paroi (114).

7. Récipient selon l'une ou plusieurs des revendications précédentes, **caractérisé par le fait que** ledit élément d'étanchéité (54) présente une ouverture de passage (115) axiale.

8. Récipient selon l'une ou plusieurs des revendications précédentes, **caractérisé par le fait que** ledit élément d'étanchéité (54) est réalisé de manière concentrique par rapport à la direction axiale (102).

9. Récipient selon l'une ou plusieurs des revendications précédentes, **caractérisé par le fait que** ledit élément d'étanchéité (54) présente un étagement (116) par rapport à la direction axiale (102) contre lequel le filtre (100) s'applique et lequel est réalisé de préférence radialement à l'intérieur.

10. Récipient selon l'une ou plusieurs des revendications précédentes, **caractérisé par le fait que** ledit élément d'étanchéité (54) présente une rallonge tubulaire (118) qui fait saillie dans le volume intérieur du récipient (8) et dans laquelle s'engage le filtre (100).

11. Récipient selon l'une ou plusieurs des revendications précédentes, **caractérisé par le fait que** ledit élément d'étanchéité (54) présente une rallonge tubulaire (120) qui fait saillie du récipient (8) en direction de la première partie de boîtier (4) et au moyen de laquelle peut être établie une communication fluidique vers le dispositif générant de la pression négative.

12. Récipient selon l'une ou plusieurs des revendications précédentes, **caractérisé par le fait qu'**un autre élément d'étanchéité (56) est prévu qui est disposé entre une chambre (48) à l'intérieur du récipient (8) et un raccord de milieu ou de mesure dans la première partie de boîtier (4) et qui fait saillie, par une rallonge tubulaire (124), du récipient (8) en direction de la première partie de boîtier (4).

13. Récipient selon la revendication 12, **caractérisé par le fait que** ledit autre élément d'étanchéité (56) est une pièce moulée par injection en un matériau élastiquement flexible qui, de préférence, est disposée dans une ouverture de passage dans une paroi de la première partie en demi-cuvette (20) du récipient (8) et est surmoulée par injection sur ladite paroi.

14. Récipient selon la revendication 12 ou 13, **caractérisé par le fait que** les éléments d'étanchéité (54, 56) sont réalisées dans la même opération de moulage par injection et sont reliées, dans le récipient (8), sur une distance de remplissage (126) remplie de matériau élastomère.

15. Procédé de fabrication du récipient selon l'une ou plusieurs des revendications précédentes, comprenant les étapes de procédé suivantes:
- fournir les deux parties en demi-cuvette (20, 22),
- surmouler par injection ledit élément d'étanchéité (54) sur la partie en demi-cuvette (20) montrant vers la première partie de boîtier (4),
- disposer le filtre (100) sur l'élément d'étanchéité (54) ou insérer le filtre (100) dans l'élément d'étanchéité (54),
- assembler les deux parties en demi-cuvette (20, 22) en interposant le filtre (100) qui, lors de cela, est plaqué dans une direction axiale (102) contre ledit élément d'étanchéité (54), et générer une tension dans la direction axiale (102) par l'assemblage des parties en demi-cuvette (20, 22) et par, allant de pair avec ce dernier, la déformation de l'élément d'étanchéité (54) par le filtre (10) plaqué contre celui-ci, et
- relier entre elles de manière inamovible les deux parties en demi-cuvette (20, 22).

16. Dispositif (2) qui peut être porté sur le corps d'un utilisateur et peut, en sus, fonctionner de manière stationnaire et qui est destiné à fournir de la pression négative pour des applications médicales, en particulier pour le traitement par pression négative de plaies sur le corps humain ou animal, comprenant un dispositif générant de la pression négative et un récipient (8) jetable après l'utilisation et destiné à recevoir dans son intérieur des liquides corporels, en particulier des exsudats aspirés d'une plaie, dans lequel ledit dispositif générant de la pression négative est disposé dans ou sur une première partie de boîtier (4) du dispositif et ledit récipient (8) forme une deuxième partie de boîtier (6) du dispositif et peut être fixé manuellement sur la première partie de boîtier (4) du dispositif et en être détaché manuellement, et, lorsqu'il est fixé, l'intérieur du récipient (8) peut être mis sous pression négative par ledit dispositif générant de la pression négative, et dans lequel un raccord (10) pour un conduit d'aspiration menant vers le corps est prévu sur le récipient (8) de sorte qu'une communication par pression négative peut être établie entre le dispositif générant de la pression négative, le récipient (8) et ledit conduit d'aspiration menant vers le corps, **caractérisé par** un récipient (8) selon l'une ou plusieurs des revendications précédentes 1 à 14.
